# EUROPEAN PATENT APPLICATION

(11) **EP 3 861 930 A1**
(43) Date of publication of application: **11.08.2021**
(21) Application number: 20155295.7
(22) Date of filing: 04.02.2020
(51) Int. Cl.: A61B 5/055, A61B 5/16, A61B 5/00, G01R 33/54, G01R 33/565

(54) **APPARATUS FOR MONITORING OF A PATIENT UNDERGOING A MAGNETIC RESONANCE IMAGE SCAN**

(71) Applicant: Koninklijke Philips N.V., 5656 AG Eindhoven (NL)
(72) Inventor: NAUTS, Sanne, 5656 AE Eindhoven (NL); WESTERINK, Joanne Henriëtte Desirée Monique, 5656 AE Eindhoven (NL); AMTHOR, Thomas Erik, 5656 AE Eindhoven (NL); SAINI, Privender Kaur, 5656 AE Eindhoven (NL); HEUVELINK-MARCK, Annerieke, 5656 AE Eindhoven (NL); VAN EE, Raymond, 5656 AE Eindhoven (NL); HEIJMAN, Edwin, 5656 AE Eindhoven (NL)
(74) Representative: Philips Intellectual Property & Standards

(57) **Abstract**

The present invention relates to an apparatus (10a) for monitoring of a patient undergoing a Magnetic Resonance Image (MRI) scan. The apparatus comprises an input unit (20a), a processing unit (30a), and an output unit (40a). The input unit is configured to provide the processing unit with at least one sensor data of a patient undergoing an MRI scan by an MRI scanner. The input unit is configured to provide the processing unit with at least one scan parameter of the MRI scanner for the MRI scan. The input unit is configured to provide the processing unit with at least one characteristic of the patient. The processing unit is configured to predict a stress level of the patient and/or a predicted motion state of the patient, the prediction or predictions comprising utilization of the at least one sensor data of the patient, the at least one scan parameter of the MRI scanner, and the at least one characteristic of the patient. The output unit is configured to output information relating to the predicted stress level of the patient and/or the predicted motion state of the patient.

## Description

### FIELD OF THE INVENTION

The present invention relates to an apparatus for monitoring of a patient undergoing a Magnetic Resonance Image (MRI) scan, an imaging system, a method for monitoring of a patient undergoing a Magnetic Resonance Image scan, as well as to a computer program element and a computer readable medium.

### BACKGROUND OF THE INVENTION

Patient cooperation is an important determinant of the duration and diagnostic quality of an MRI-exam. To acquire an MR image, patients are required to enter a narrow bore in an unfamiliar loud machine and lie still for approximately 15 to 60 minutes. As such, many patients experience anxiety-related reactions to an MRI-exam (Melendez, J. C., & McCrank, E. (1993). Anxiety-related reactions associated with magnetic resonance imaging examinations. Jama, 270(6), 745-747), and patient movement is highly common in MRI-exams (occurring in 7% to 29% of MRI examinations; Andre, J. B., Bresnahan, B. W., Mossa-Basha, M., Hoff, M. N., Smith, C. P., Anzai, Y., & Cohen, W. A. (2015). Toward quantifying the prevalence, severity, and cost associated with patient motion during clinical MR examinations. Journal of the American College of Radiology, 12(7), 689-695). Patient movement may lead to the need to repeat parts of the MRI-exam (appr. 20% of MRI-scans is a repeat scan; Andre et al., 2015). Moreover, patients who are in severe discomfort sometimes leave the scanner before the exam is finished (these are called "incompleted exams").

Even though this anxiety can be detrimental for MRI success, currently hospital staff have very limited information about a participant's psychological state (e.g., level of anxiety or discomfort). They can only assess the mental state of the patient, their stress levels and therefore the likelihood of movement artefacts, by means of standard interpersonal contact. Moreover, they can only do so just before the actual scanning.

Thus the emotional and physiological state of the patient is not easily visible or available to the technologist during an MRI examination or scan. However, if a patient becomes very anxious or feels pain, or moves unduly the scan should be stopped. In some cases, the patient may press the alarm button or make him/herself heard acoustically. But the decision to press the button can be very late. In the worst case, the button may not work due to a technical problem, the button could be lost or out of contact by the patient, or the patient may have lost consciousness without the technologist noticing.

Therefore, patient motion during the acquisition of an MRI image is a dominant factor in having artefacts within the MRI images. These artefacts not only lead to deterioration of image quality, but also result in reduction of the diagnostic value, however it is very hard to compensate for sudden and deformable body motion during an MR acquisition. To compensate for this, MRI scans are often repeated, which considerably increases the examination time. Even fine body motion during a short period leads to motion artefacts in MR images. Therefore, technology has been developed to reduce these motion artefacts (e.g. MultiVane sequences, etc.) or detect them and remove them from the raw data or during image reconstruction. However, scan time is lost when the motion becomes visible in the image after the reconstruction as this data is not viable. Also, motion during a contrast agent injection cannot be repeated in the same examination due to the decay time of the agent.

There is a need to address these issues.

### SUMMARY OF THE INVENTION

It would be advantageous to have improved means of determining when to stop and/or intervene in an MRI scan due to the patient becoming too anxious or moving body parts too much. The object of the present invention is solved with the subject matter of the independent claims, wherein further embodiments are incorporated in the dependent claims. It should be noted that the following described aspects and examples of the invention apply also to the apparatus or monitoring of a patient undergoing a Magnetic Resonance Image scan, the imaging system, the method for monitoring of a patient undergoing a Magnetic Resonance Image scan, as well as to the computer program element and a computer readable medium.

In a first aspect, there is provided an apparatus for monitoring of a patient undergoing a Magnetic Resonance Image (MRI) scan, the apparatus comprising:
- an input unit;
- a processing unit; and
- an output unit.

The input unit is configured to provide the processing unit with at least one sensor data of a patient undergoing an MRI scan by an MRI scanner. The input unit is configured to provide the processing unit with at least one scan parameter of the MRI scanner for the MRI scan. The processing unit is configured to predict if an intervention to the MRI scan and/or MRI scanner and/or patient and/or operator of the MRI scanner is required, the prediction comprising utilization of the at least one sensor data of the patient, and the at least one scan parameter of the MRI scanner. The output unit is configured to output information relating to the prediction of the intervention.

In an example, the input unit is configured to provide the processing unit with at least one characteristic of the patient. The prediction if an intervention to the MRI scan and/or MRI scanner and/or patient and/or operator of the MRI scanner is required can then comprise a prediction of a motion state of the patient. The prediction of the motion state can comprise utilization of the at least one sensor data of the patient, the at least one scan parameter of the MRI scanner, and the at least one characteristic of the patient.

In an example, the at least one characteristic of the patient comprises one or more of: prior movement data for the patient, age, weight, body-mass index, information on a previous diagnosis, physical condition of the patient, psychological condition of the patient, completed questionnaire information, patient feedback.

In an example, the prior movement data for the patient comprises information regarding one or more previous MRI scans undertaken by the patient.

In an example, the processing unit is configured to implement at least one machine learning algorithm to predict the motion state of the patient. The at least one machine learning algorithm can be trained on the basis of; at least one sensor data of one or more reference patients undergoing one or more reference MRI scans by one or more reference MRI scanners; at least one scan parameter of the reference MRI scanners used for the one or more reference MRI scans.

In an example, the at least one sensor data of the patient was acquired by one or more of: camera, microphone, skin resistance sensor, skin temperature sensor, skin humidity sensor, skin accelerometer, skin colour sensor, skin conduction sensor, pulse sensor, breathing sensor, Oxygen saturation sensor, radio frequency radar sensor, EEG sensor, ECG sensor, pressure sensor, weight sensor.

In an example, the at least one sensor data of the one or more reference patients was acquired by one or more of: camera, microphone, skin resistance sensor, skin temperature sensor, skin humidity sensor, skin accelerometer, skin colour sensor, skin conduction sensor, pulse sensor, breathing sensor, Oxygen saturation sensor, radio frequency radar sensor, EEG sensor, ECG sensor, pressure sensor, weight sensor.

In an example, the at least one scan parameter of the MRI scanner comprises one or more of: duration of scan, duration of scan remaining, current gradient strengths, future gradient strengths, type of contrast, timing parameters, SAR (RF-settings), k-space sampling pattern, coil or coils used, number of averages, EPI factor, resolution, scan plane, motion direction, anatomic region being scanned.

In an example, the at least one scan parameter of the reference MRI scanners comprises one or more of: duration of scan, duration of scan remaining, current gradient strengths, future gradient strengths, type of contrast, timing parameters, SAR (RF-settings), k-space sampling pattern, coil or coils used (e.g., knee coil, head coil, etc.), number of averages, EPI factor, resolution, scan plane, motion direction, anatomic region being scanned.

In an example, the at least one characteristic of each of the reference patient comprises one or more of: prior movement data for the patient, age, weight, body-mass index, information on a previous diagnosis, physical condition of the patient, psychological condition of the patient, completed questionnaire information.

In an example, training of the machine learning algorithm can comprise utilization of information regarding one or more previous MRI scans undertaken by at least one reference patient of the one or more reference patients.

In an example, the processing unit is configured to determine if the predicted motion state of the patient will exceed a motion threshold level. The prediction if an intervention to the MRI scan and/or MRI scanner and/or patient and/or operator of the MRI scanner is required can comprise a determination if a movement level of the patient is predicted to exceed the threshold.

In an example, the motion threshold level is determined on the basis of at least one remaining scan sequence for the MRI scan.

In a second aspect, there is provided an imaging system comprising;
- a magnetic resonance imaging (MRI) scanner;
- at least one sensor; and
- an apparatus for monitoring of a patient undergoing a Magnetic Resonance Image (MRI) scan according to the first aspect.

The at least one sensor is configured to provide the processing unit of the apparatus with at least one sensor data of a patient undergoing an MRI scan by an MRI scanner. The apparatus is configured to automatically provide an intervention to the MRI scan and/or MRI scanner and/or patient and/or operator of the MRI scanner on the basis of the information relating to the prediction of the intervention.

In a third aspect, there is provided a method for monitoring of a patient undergoing a Magnetic Resonance Image (MRI) scan, the method comprising:
- providing a processing unit with at least one sensor data of a patient undergoing an MRI scan by an MRI scanner;
- providing the processing unit with at least one scan parameter of the MRI scanner for the MRI scan;
- predicting by the processing unit if an intervention to the MRI scan and/or MRI scanner and/or patient and/or operator of the MRI scanner is required, the prediction comprising utilization of the at least one sensor data of the patient, and the at least one scan parameter of the MRI scanner; and
- outputting by an output unit information relating to the prediction of the intervention.

According to another aspect, there is provided a computer program element controlling one or more of the apparatuses or system as previously described which, if the computer program element is executed by a processing unit, is adapted to perform one or more of the methods as previously described.

According to another aspect, there is provided a computer readable medium having stored computer element as previously described.

The computer program element can for example be a software program but can also be a FPGA, a PLD or any other appropriate digital means.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawing:
Fig. 1 shows a schematic set up of an example of an apparatus for monitoring of a patient undergoing a Magnetic Resonance Image scan;
Fig. 2 shows a schematic set up of an example of an imaging system;
Fig. 3 shows a method for monitoring of a patient undergoing a Magnetic Resonance Image scan;
Fig. 4 shows a high level system architecture of a detailed embodiment of the apparatus of Fig. 1 or imaging system of Fig. 2 having such an apparatus;
Fig. 5 shows examples of stress level indicators;
Fig. 6 shows a schematic set up of an example of an apparatus for monitoring of a patient undergoing a Magnetic Resonance Image scan;
Fig. 7 shows a schematic set up of an example of an imaging system;
Fig. 8 shows a method for monitoring of a patient undergoing a Magnetic Resonance Image scan;
Fig. 9 shows a high level system architecture of a detailed embodiment of the apparatus of Fig. 6 or imaging system of Fig. 7 having such an apparatus;
Fig. 10 shows examples of motion level indicators;
Fig. 11 shows an overview of data input, anxiety or stress measurement and prediction and movement measurement and prediction, and intervention as provided within the apparatus of Fig. 6 or imaging system of Fig. 7 having such an apparatus;
Fig. 12 shows a high level architecture of an apparatus of Fig. 6 or imaging system of Fig. 7 having such an apparatus; and
Fig. 13 shows a representation of an MR sequence, pre-defined tolerable motion levels, and measured actual and predicted motion level as functions of time.

### DETAILED DESCRIPTION OF EMBODIMENTS

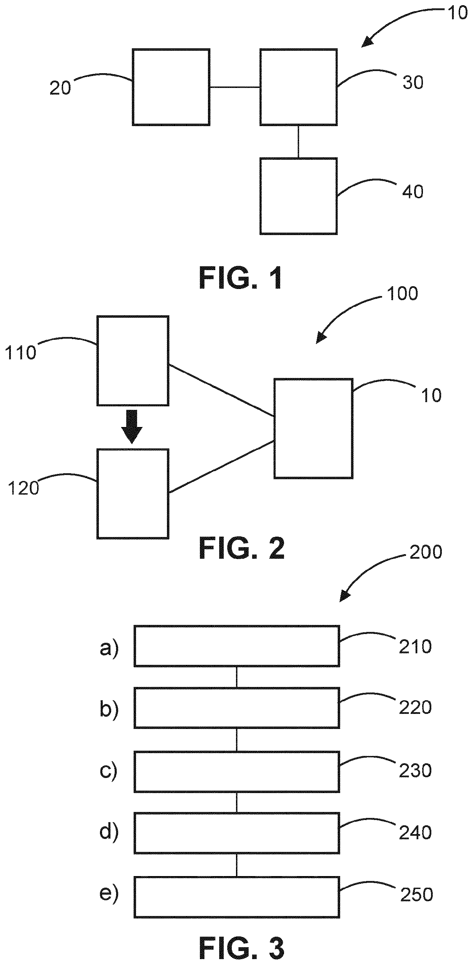

Fig. 1 shows an example of an apparatus 10 for monitoring of a patient undergoing a Magnetic Resonance Image (MRI) scan. The apparatus 10 comprises an input unit 20, a processing unit 30, and an output unit 40. The input unit is configured to provide the processing unit with at least one sensor data of a patient undergoing an MRI scan by an MRI scanner. The input unit is configured also to provide the processing unit with at least one scan parameter of the MRI scanner for the MRI scan. The input unit is configured also to provide the processing unit with at least one characteristic of the patient. The processing unit is configured to predict a stress level of the patient and/or predict a motion state of the patient, the prediction or predictions comprising utilization of the at least one sensor data of the patient, the at least one scan parameter of the MRI scanner, and the at least one characteristic of the patient. The output unit is configured to output information relating to the predicted stress level of the patient and/or the predicted motion state of the patient.

In this manner, a patients' stress level and/or likelihood of movement is determined by analyzing the emotional and physiological state of the patient from sensor data, data about the scan being conducted, and from data about the patient and the development of these states is predicted into the future. Thus, real-time feedback can be provided to the technologist, who can then decides that a scan should be stopped and indeed the apparatus can automatically initiated such a stop if it is predicted that the patient is about to enter an anxiety state or movement state that is not consistent with the scan protocol.

Thus, the apparatus objectively measures and predicts a patient's level of anxiety/discomfort during scanning, as well as the predicted likelihood of movement artefacts. This information can be used to abort a scan automatically if required.

The issues addressed by the apparatus are that patient anxiety/discomfort is mitigated, which may otherwise negatively affect patients' experience. Scans can be stopped before patient motion, which is associated with reduced image quality, has compromised image quality.

A motion state of the patient can also be termed a movement state of the patient.

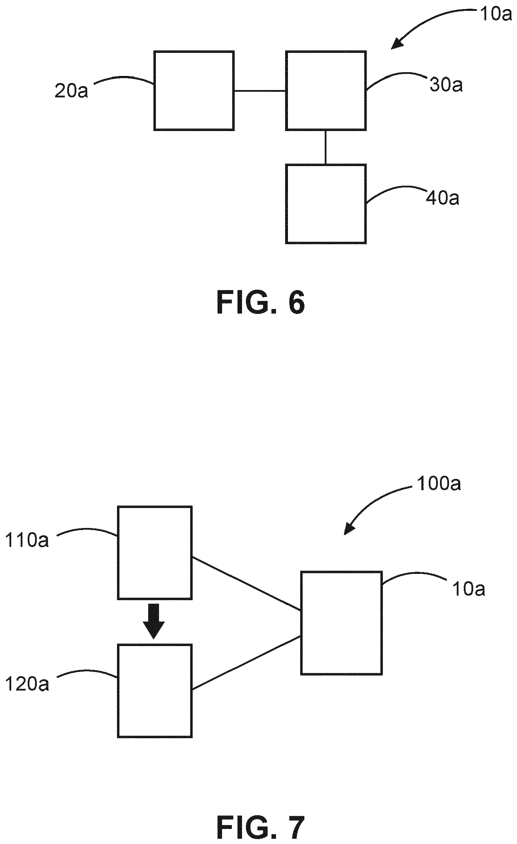

As discussed in more detail below with respect to Figs. 6-13, determining a stress state and/or motion state and utilization of the at least one characteristic of the patient is not essentially required when determining

In an example, the processing unit is configured to implement at least one machine learning algorithm to predict the stress level of the patient and/or the motion state of the patient. The at least one machine learning algorithm was trained on the basis of: at least one sensor data of one or more reference patients undergoing one or more reference MRI scans by one or more reference MRI scanners; at least one scan parameter of the reference MRI scanners used for the one or more reference MRI scans; at least one characteristic of each of the reference patients.

Thus, an AI-based algorithm is used to infer the emotional and physical state of a patient from sensor data, scan protocol data, and context information relating to the patient, enabling the development of the stress state and the movement state of the patient to be predicted.

In other words, a prediction algorithm takes data about a patient acquired before an exam that can be gathered at a hospital or at home and uses this with information gathered from sensors during a scan as well as details about the scan to predict the patient's anxiety level and movement level during the scan, where this determination can also make use of results of previous examinations.

In an example, the machine learning algorithm comprises one or more neural networks.

In an example, the machine learning algorithm comprises one or more classical machine learning algorithms.

In an example, the machine learning algorithm comprises one or more Support Vector Machine (SVM).

In an example, the machine learning algorithm comprises one or more decision Trees.

In an example, the at least one machine learning algorithm comprises two parts. A first part of the machine learning algorithm is used to determine a stress state and movement state of a person. Here movement state can mean a likelihood of moving, which can have different levels even for a stationary patient. Thus, a stationary patient can be determined to have a low likelihood of moving, or a stationary patient can be determined to have a high likelihood of moving. A second part of the machine learning algorithm can then operate to determine a prediction into the future of the stress state and movement state of the person. The first part can be for example be a standard neural network such as a convolutional neural network (CNN), and the second part can be a recurrent neural network (RNN) or long short-term memory (LSTM) neural network version of a RNN. Thus, here neural network can refer to a combination of neural networks.

In an example, the at least one sensor data of the patient was acquired by one or more of: camera, microphone, skin resistance sensor, skin temperature sensor, skin humidity sensor, skin accelerometer, pulse sensor, breathing sensor, radio frequency radar sensor, EEG sensor, processing unit, pressure sensor, weight sensor.

In an example, the at least one sensor data of the one or more reference patients was acquired by one or more of: camera, microphone, skin resistance sensor, skin temperature sensor, skin humidity sensor, skin accelerometer, pulse sensor, breathing sensor, radio frequency radar sensor, EEG sensor, processing unit, pressure sensor, weight sensor

In an example, the at least one sensor data of the patient comprises one or more of: breathing rate data, heart rate data, voice data, skin resistance data, skin temperature data, skin humidity data, skin motion data, body part movement data, blinking frequency data, EEG data, information relating to what is being shown on an in-bore display, weight distribution on in-bore examination table.

In an example, the at least one sensor data of the one or more reference patients comprises one or more of: breathing rate data, heart rate data, voice data, skin resistance data, skin temperature data, skin humidity data, skin motion data, body part movement data, blinking frequency data, EEG data, information relating to what is being shown on an in-bore display, weight distribution on in-bore examination table.

In an example, the at least one scan parameter of the MRI scanner comprises one or more of: duration of scan, duration of scan remaining, current gradient strengths, future gradient strengths, type of contrast, timing parameters, SAR (RF-settings), k-space sampling pattern.

In an example, the at least one scan parameter of the reference MRI scanners comprises one or more of: duration of scan, duration of scan remaining, current gradient strengths, future gradient strengths, type of contrast, timing parameters, SAR (RF-settings), k-space sampling pattern.

In an example, the at least one characteristic of the patient comprises one or more of: age, weight, body-mass index, information on a previous diagnosis, physical condition of the patient, psychological condition of the patient, completed questionnaire information, patient feedback.

In an example, the at least one characteristic of each of the reference patient comprises one or more of: age, weight, body-mass index, information on a previous diagnosis, physical condition of the patient, psychological condition of the patient, completed questionnaire information.

In an example, the input unit is configured to provide the processing unit with information regarding one or more previous MRI scans undertaken by the patient. The prediction of the stress level of the patient and/or the prediction of the motion state of the patient can then comprise utilization of the information regarding one or more previous MRI scans undertaken by the patient.

In an example, training of the machine learning algorithm comprises utilization of information regarding one or more previous MRI scans undertaken by at least one reference patient of the one or more reference patients.

In an example, the processing unit is configured to determine if the predicted stress level of the patient and/or predicted motion state of the patient will exceed a stress threshold level or a motion threshold level. The information relating to the predicted stress level of the patient and/or the predicted motion state of the patient can then comprise an indication if one or other or both of these thresholds is predicted to be exceeded.

Fig. 2 shows an example of an imaging system 100. The imaging system 100 comprises a magnetic resonance imaging scanner 110, at least one sensor 120. The system 100 also comprises an apparatus 10 for monitoring of a patient undergoing a Magnetic Resonance Image (MRI) scan as described with respect to Fig. 2. The at least one sensor 120 is configured to provide the processing unit of the apparatus with at least one sensor data of a patient undergoing an MRI scan by an MRI scanner. The apparatus 10 is configured to automatically stop the MRI scan being performed by the magnetic resonance imaging scanner 110 on the basis of the information relating to the predicted stress level of the patient and/or the predicted motion state of the patient.

Fig. 3 shows a method 200 for monitoring of a patient undergoing a Magnetic Resonance Image (MRI) scan in its basic steps. The method 200 comprises:
- in a providing step 210, also referred to as step a), providing a processing unit with at least one sensor data of a patient undergoing an MRI scan by an MRI scanner;
- in a providing step 220, also referred to as step b), providing the processing unit with at least one scan parameter of the MRI scanner for the MRI scan;
- in a providing step 230, also referred to as step c), providing the processing unit with at least one characteristic of the patient;
- in a predicting step 240, also referred to as step d), predicting by the processing unit a stress level of the patient and/or predicting a motion state of the patient, the prediction or predictions comprising utilization of the at least one sensor data of the patient, the at least one scan parameter of the MRI scanner, and the at least one characteristic of the patient; and
- in an outputting step 250, also referred to as step e), outputting by an output unit information relating to the predicted stress level of the patient and/or the predicted motion state of the patient.

In an example, the machine learning algorithm comprises one or more neural networks.

In an example, the machine learning algorithm comprises one or more classical machine learning algorithms.

In an example, the machine learning algorithm comprises one or more Support Vector Machine).

In an example, the machine learning algorithm comprises one or more decision Trees.

In an example, step d) comprises implementing by the processing unit at least one machine learning algorithm to predict the stress level of the patient and/or the motion state of the patient, wherein the at least one machine learning algorithm was trained on the basis of one or more of: at least one sensor data of one or more reference patients undergoing one or more reference MRI scans by one or more reference MRI scanners; at least one scan parameter of the reference MRI scanners used for the one or more reference MRI scans; at least one characteristic of each of the reference patients.

In an example, the at least one sensor data of the patient was acquired by one or more of: camera, microphone, skin resistance sensor, skin temperature sensor, skin humidity sensor, skin accelerometer, pulse sensor, breathing sensor, radio frequency radar sensor, EEG sensor, processing unit, pressure sensor, weight sensor.

In an example, the at least one sensor data of the one or more reference patients was acquired by one or more of: camera, microphone, skin resistance sensor, skin temperature sensor, skin humidity sensor, skin accelerometer, pulse sensor, breathing sensor, radio frequency radar sensor, EEG sensor, processing unit, pressure sensor, weight sensor

In an example, the at least one sensor data of the patient comprises one or more of: breathing rate data, heart rate data, voice data, skin resistance data, skin temperature data, skin humidity data, skin motion data, body part movement data, blinking frequency data, EEG data, information relating to what is being shown on an in-bore display, weight distribution on in-bore examination table.

In an example, the at least one sensor data of the one or more reference patients comprises one or more of: breathing rate data, heart rate data, voice data, skin resistance data, skin temperature data, skin humidity data, skin motion data, body part movement data, blinking frequency data, EEG data, information relating to what is being shown on an in-bore display, weight distribution on in-bore examination table.

In an example, the at least one scan parameter of the MRI scanner comprises one or more of: duration of scan, duration of scan remaining, current gradient strengths, future gradient strengths, type of contrast, timing parameters, SAR (RF-settings), k-space sampling pattern.

In an example, the at least one scan parameter of the reference MRI scanners comprises one or more of: duration of scan, duration of scan remaining, current gradient strengths, future gradient strengths, type of contrast, timing parameters, SAR (RF-settings), k-space sampling pattern.

In an example, the at least one characteristic of the patient comprises one or more of: age, weight, body-mass index, information on a previous diagnosis, physical condition of the patient, psychological condition of the patient, completed questionnaire information, patient feedback.

In an example, the at least one characteristic of each of the reference patient comprises one or more of: age, weight, body-mass index, information on a previous diagnosis, physical condition of the patient, psychological condition of the patient, completed questionnaire information.

In an example, the method comprises providing the processing unit with information regarding one or more previous MRI scans undertaken by the patient, and wherein step d) comprises utilizing the information regarding one or more previous MRI scans undertaken by the patient.

In an example, training of the machine learning algorithm comprised utilization of information regarding one or more previous MRI scans undertaken by at least one reference patient of the one or more reference patients.

In an example, step d) comprises determining by the processing unit if the predicted stress level of the patient and/or predicted motion state of the patient will exceed a stress threshold level or a motion threshold level, and wherein in step e) the information relating to the predicted stress level of the patient and/or the predicted motion state of the patient comprises an indication if either threshold is predicted to be exceeded.

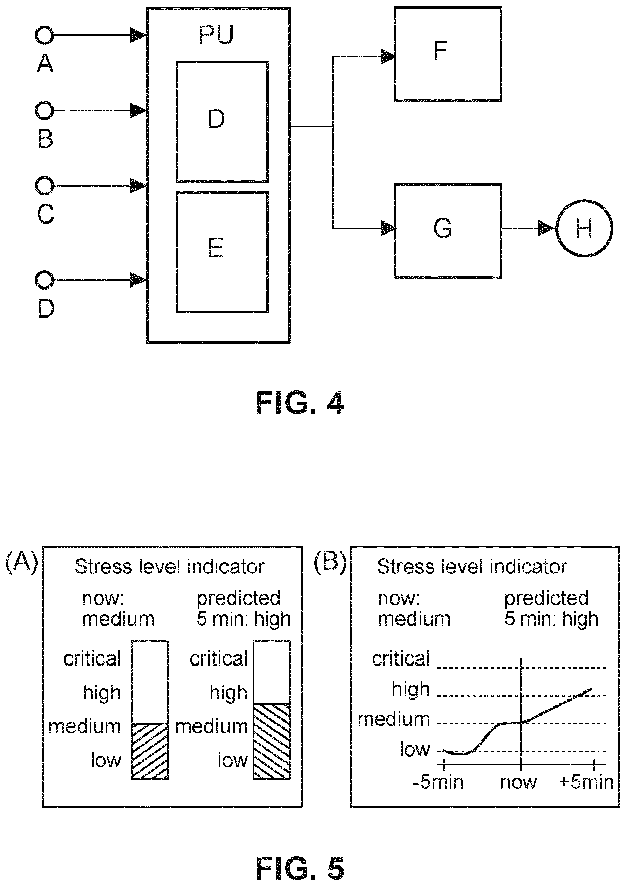

The apparatus for monitoring of a patient undergoing a Magnetic Resonance Image scan, the imaging system, and the method for monitoring of a patient undergoing a Magnetic Resonance Image scan are now described in further detail with respect to specific embodiments, where reference is made to Figs. 4-5. Here, specific relevance relates to stress detection and motion detection, and preventing these from becoming problematic by stopping the scan process if required.

Fig. 4 shows a high-level system architecture. A patient is undergoing an MRI scan, and biometric and physiological sensors indicated at "A" provide corresponding data to the processing unit indicated out "PU". Scan parameters for the MRI scan, indicated at "B", also provided to the processing unit. Here, the structure of the ExamCard can also be provided to the processing unit, including information such as how long the complete exam or scan will take and how much time is remaining. Information relating to the patient, indicated at "C", is also provided to the processing unit. The processing unit implements a first neural network, indicated at "D", such as a convolutional neural network to predict a stress level for the patient and predict of movement state level of the patient. The processing unit also implements a second new network, indicated at "E", such as a recurrent neural network or Long Short term memory comes new network that acts to predict into the future the stress level and movement state level of the patient. The current and predicted stress level is then displayed to the operator, indicated at "F", and emergency decision logic indicated at "G", utilises the predicted stress level and the predicted movement state level to determine if an emergency stop to the scan should be made, indicated at "H". Thus, the processing unit predicts the patient's stress level and movement state based on a number of parameters ("features") obtained from sensors, scan settings, and patient information. Patient feedback on the experienced stress and movement for that patient and other patients can also be provided for training the internal machine learning algorithms, which can be combined with sensor data and scan parameter information and patient information for those patients undergoing those scans as part of that training.

Fig. 5 shows two examples of stress level indicators determined by the processing unit that are presented to the operator or technologist operating the MRI scanner, indicating the present stress level and future stress level and how that stress level has been progressing up to now and how it is predicted to progress into the future. Motion (or movement) state level indicators, indicating the likelihood of movement of the patient as of now, in the past, and into the future can also be provided in a similar manner. The stress level information and/or movement state level information enables the technologist or operator to initiate an emergency stop if required, and the system itself also can initiate the emergency stop if it is predicted that the stress level will go too high or the likelihood of the patient moving is too great.

The emergency detection logic operates based on the current and predicted stress level and on the current and predicted movement state level information, where the emergency detection logic decides when to stop the MRI scan. Ideally, the logic is configured in a way that the scan is stopped when an increase of stress level above the critical level is expected while the scan is running, or when the likelihood of the patient moving to an unacceptable level is expected, which would mean that the patient would otherwise press the emergency button with a high probability or move in a manner that would compromise scanning image integrity or result in a potentially dangerous situation arising.

Fig. 6 shows an example of an apparatus 10a for monitoring of a patient undergoing a Magnetic Resonance Image (MRI) scan. The apparatus comprises an input unit 20a, a processing unit 30a, and an output unit 40a. The input unit is configured to provide the processing unit with at least one sensor data of a patient undergoing an MRI scan by an MRI scanner. The input unit is configured to provide the processing unit with at least one scan parameter of the MRI scanner for the MRI scan. The processing unit is configured to predict if an intervention to the MRI scan and/or MRI scanner and/or patient and/or operator of the MRI scanner is required, the prediction comprising utilization of the at least one sensor data of the patient, and the at least one scan parameter of the MRI scanner. The output unit is configured to output information relating to the prediction of the intervention.

In an example the prediction if an intervention to the MRI scan and/or MRI scanner and/or patient and/or operator of the MRI scanner is required comprises information on the intervention to be applied.

Thus, not only can a determination be made based on sensor data and scan parameters that an intervention is required, to mitigate image degradation or the patient about to endanger themselves for example, but the apparatus based on these parameters can determine the type of intervention to be made.

In an example, the predicted intervention comprises an intervention applied to the MRI scan, such as stopping or aborting the scan, or changing scan parameters such as resolution, or changing a scan sequence in order that a quieter scan sequence is scheduled next rather than a planned noisy scan sequence.

In an example, the predicted intervention comprises an intervention applied to the patient, such as coaching the patient, reassuring the patient, asking the patient to keep still or keep a part of their body still, changing what patients see on the in-bore screen.

In an example, the predicted intervention comprises an intervention applied to the operator, such as indicating that the MRI scan should be changed in some manner, or that a specific intervention needs to be applied to the patient, or indicating that the patient is likely to compromise image quality in the near future and the operator can determine what action to take.

In an example, the predicted intervention comprises a prediction of a time to provide the intervention.

Thus for example, the information relating to the predicted time to provide an intervention to the patient can be provided to an operator of the MRI scanner, who can then provide an appropriate intervention to the patient, such as to please stop moving and this can relate to a part of the patient's body.

Thus for an example, the information relating to the predicted time to provide an intervention to the patient can be provided automatically to the patient. Thus, for example, a screen or VDU the patient can see can, at an appropriate time, indicate to the patient that they are beginning to move in a manner that should be stopped.

Thus, the patient can be provided with information to limit their movement before it leads to (further) image degradation, and this is only provided when necessary. Thus, an intervention can be made prior to any image degradation having occurred, or an intervention can be made where some image degradation has taken place but the apparatus prevents further degradation from occurring.

In an example, the predicted intervention comprises a change to video and/or music the patient is watching or listening to. Thus, to help the patient to limit their movements, for a patient who is presented with in bore (their favourite) music or video image, is to tell them that the video/music quality will be reduced (e.g. by adding noise or by high-or low-pass filtering) if they move too much and/or automatically reducing the image quality as their movement level increases and increasing the quality as their movement level reduces. This can be formed in an automatic feedback loop that motivates and reminds the patients in a natural way to reduce their movements.

According to an example, the input unit is configured to provide the processing unit with at least one characteristic of the patient. The prediction if an intervention to the MRI scan and/or MRI scanner and/or patient and/or operator of the MRI scanner is required can comprise a prediction of a motion state of the patient. The prediction of the motion state can comprise utilization of the at least one sensor data of the patient, the at least one scan parameter of the MRI scanner, and the at least one characteristic of the patient.

According to an example, the at least one characteristic of the patient comprises one or more of: prior movement data for the patient, age, weight, body-mass index, information on a previous diagnosis, physical condition of the patient, psychological condition of the patient, completed questionnaire information, patient feedback.

According to an example, the prior movement data for the patient comprises information regarding one or more previous MRI scans undertaken by the patient.

In an example, the prior movement data for the patient comprises information relating to a prior MRI survey scan carried out immediately prior to the MRI scan that the patient is undergoing.

In an example, the prior movement data for the patient comprises information relating to a prior different MRI scan carried out to the MRI scan that the patient is undergoing, for example, one week, one month or one month before.

In an example, the prior movement data for the patient comprises information relating to a prior scan sequence of the MRI scan that the patient is undergoing. Thus, for example the prior movement data can some from a previous sequence within the same examcard (that could have carried out a minute earlier).

According to an example, the processing unit is configured to implement at least one machine learning algorithm to predict the motion state of the patient. The at least one machine learning algorithm can be trained on the basis of; at least one sensor data of one or more reference patients undergoing one or more reference MRI scans by one or more reference MRI scanners; at least one scan parameter of the reference MRI scanners used for the one or more reference MRI scans.

In an example, the at least one machine learning algorithm was trained on the basis of; at least one characteristic of each of the reference patients

Thus, an AI-based algorithm is used to infer the emotional and physical state of a patient from sensor data, scan protocol data, and can also take account of context information relating to the patient, enabling the development of the movement state of the patient to be predicted.

In other words, a prediction algorithm can take data about a patient acquired before an exam that can be gathered at a hospital or at home and uses this with information along with data gathered from sensors during a scan as well as details about the scan to predict the patient's movement level during the scan, where this determination can also make use of results of previous examinations.

In an example, the machine learning algorithm comprises one or more neural networks.

In an example, the machine learning algorithm comprises one or more classical machine learning algorithms.

In an example, the machine learning algorithm comprises one or more Support Vector Machine (SVM).

In an example, the machine learning algorithm comprises one or more decision Trees.

In an example, the at least one machine learning algorithm comprises two parts. A first part of the machine learning algorithm is used to determine a movement state of a person. Here movement state can mean a likelihood of moving, which can have different levels even for a stationary patient. Thus, a stationary patient can be determined to have a low likelihood of moving, or a stationary patient can be determined to have a high likelihood of moving. A second part of the machine learning algorithm can then operate to determine a prediction into the future of the movement state of the person. The first part can be for example be a standard neural network such as a convolutional neural network (CNN), and the second part can be a recurrent neural network (RNN) or long short-term memory (LSTM) neural network version of a RNN. Thus, here neural network can refer to a combination of neural networks.

According to an example, the at least one sensor data of the patient was acquired by one or more of: camera, microphone, skin resistance sensor, skin temperature sensor, skin humidity sensor, skin accelerometer, skin colour sensor, skin conduction sensor, pulse sensor, breathing sensor, Oxygen saturation sensor, radio frequency radar sensor, EEG sensor, ECG sensor, pressure sensor, weight sensor.

In an example, a Galvanic Skin Response sensor can be utilized to provide skin humidity and skin conductance data.

Thus, one or more sensors can be used to determine sensor data for the patient that can include electrodermal activity of the patient and this information can be utilized in determining if an intervention is required and what type of intervention is required.

According to an example, the at least one sensor data of the one or more reference patients was acquired by one or more of: camera, microphone, skin resistance sensor, skin temperature sensor, skin humidity sensor, skin accelerometer, skin colour sensor, skin conduction sensor, pulse sensor, breathing sensor, Oxygen saturation sensor, radio frequency radar sensor, EEG sensor, ECG sensor, pressure sensor, weight sensor.

In an example, the at least one sensor data of the patient comprises one or more of; breathing rate data, heart rate data, voice data, skin resistance data, skin temperature data, skin humidity data, skin motion data, body part movement data, blinking frequency data, EEG data, information relating to what is being shown on an in-bore display, weight distribution on in-bore examination table.

In an example, the at least one sensor data of the one or more reference patients comprises one or more of; breathing rate data, heart rate data, voice data, skin resistance data, skin temperature data, skin humidity data, skin motion data, body part movement data, blinking frequency data, EEG data, information relating to what is being shown on an in-bore display, weight distribution on in-bore examination table.

According to an example, the at least one scan parameter of the MRI scanner comprises one or more of: duration of scan, duration of scan remaining, current gradient strengths, future gradient strengths, type of contrast, timing parameters, SAR (RF-settings), k-space sampling pattern, coil or coils used, number of averages, EPI factor, resolution, scan plane, motion direction, anatomic region being scanned.

The coil or coils can be a knee coil or head coil for example.

According to an example, the at least one scan parameter of the reference MRI scanners comprises one or more of: duration of scan, duration of scan remaining, current gradient strengths, future gradient strengths, type of contrast, timing parameters, SAR (RF-settings), k-space sampling pattern, coil or coils used (e.g., knee coil, head coil, etc.), number of averages, EPI factor, resolution, scan plane, motion direction, anatomic region being scanned.

According to an example, the at least one characteristic of each of the reference patient comprises one or more of: prior movement data for the patient, age, weight, body-mass index, information on a previous diagnosis, physical condition of the patient, psychological condition of the patient, completed questionnaire information.

According to an example, training of the machine learning algorithm comprises utilization of information regarding one or more previous MRI scans undertaken by at least one reference patient of the one or more reference patients.

In an example, the training of the machine learning algorithm includes information of what an expert operator or clinician has determined to be an appropriate intervention.

Thus, not only can it be determined if an intervention is required and predict when it should be applied, but the type of intervention to be applied, whether to the scanner, the scan, the patient or the operator or a combination of these can also be determined.

According to an example, the processing unit is configured to determine if the predicted motion state of the patient will exceed a motion threshold level. The prediction if an intervention to the MRI scan and/or MRI scanner and/or patient and/or operator of the MRI scanner is required can comprises a determination if a movement level of the patient is predicted to exceed the threshold.

In an example, the prediction comprises a prediction of a time in the future when the movement level is expected to exceed the threshold.

Thus the threshold level can be set below that when movement will compromise imaged quality, and as the patient's movements approach the image degradation threshold the movement threshold will be reached first and the patient can be warned to stop moving before image degradation occurs.

According to an example, the motion threshold level is determined on the basis of at least one remaining scan sequence for the MRI scan.

In this manner, the threshold can take into account the actual next imaging protocol, where if the next imaging sequence is particularly sensitive to movement the movement threshold can be lowered, whilst if the next imaging steps are not particularly movement sensitive the threshold can be raised. This ensures that the patient is only notified to limit movement when absolutely necessary.

In an example, the motion threshold level can be utilized to change the quality of video and/or music being viewed/listened to by the patient.

Fig. 7 shows an example of an imaging system 100a comprising a magnetic resonance imaging (MRI) scanner 110a, at least one sensor 120a and an apparatus 10a for monitoring of a patient undergoing a Magnetic Resonance Image (MRI) scan as described with respect to Fig. 6. The at least one sensor is configured to provide the processing unit of the apparatus with at least one sensor data of a patient undergoing an MRI scan by an MRI scanner. The apparatus is configured to automatically provide an intervention to the MRI scan and/or MRI scanner and/or patient and/or operator of the MRI scanner on the basis of the information relating to the prediction of the intervention.

In an example, the imaging system comprises at least one intervention unit, configured to provide an indication to the patient to limit movement of at least one part of their body and/or head.

The at least part of the body can be a head, a chest, an arm, a leg, for example.

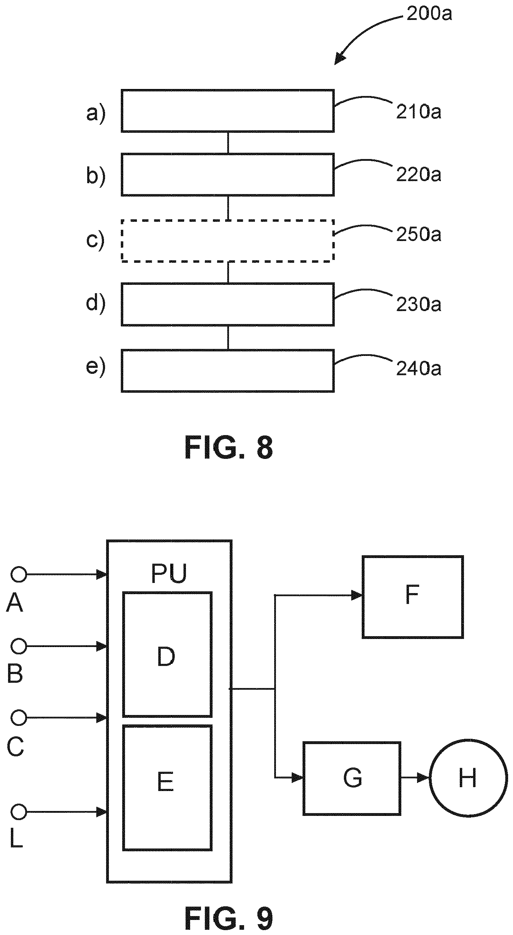

Fig. 8 shows a method 200a for monitoring of a patient undergoing a Magnetic Resonance Image (MRI) scan in its basic steps, where essential steps are shown in solid boxes and optional steps are shown in dashed boxes. The method comprises:
- in a providing step 210, also referred to as step a), providing a processing unit with at least one sensor data of a patient undergoing an MRI scan by an MRI scanner;
- in a providing step 220, also referred to as step b), providing the processing unit with at least one scan parameter of the MRI scanner for the MRI scan;
- in a predicting step 230, also referred to as step d), predicting by the processing unit if an intervention to the MRI scan and/or MRI scanner and/or patient and/or operator of the MRI scanner is required, the prediction comprising utilization of the at least one sensor data of the patient, and the at least one scan parameter of the MRI scanner; and
- in an outputting step 240, also referred to as step e), outputting by an output unit information relating to the prediction of the intervention.

In an example, the method comprises:
in a providing step 250, also referred to as step c), providing the processing unit with at least one characteristic of the patient; and wherein step d) comprises predicting a motion state of the patient comprising utilizing the at least one sensor data of the patient, the at least one scan parameter of the MRI scanner, and the at least one characteristic of the patient.

In an example, the at least one characteristic of the patient comprises one or more of: prior movement data for the patient, age, weight, body-mass index, information on a previous diagnosis, physical condition of the patient, psychological condition of the patient, completed questionnaire information, patient feedback.

In an example, the prior movement data for the patient comprises information regarding one or more previous MRI scans undertaken by the patient.

In an example, the prior movement data for the patient comprises information relating to a priorMRI survey scan carried out immediately prior to the MRI scan that the patient is undergoing.

In an example, step d) comprises implementing by the processing unit at least one machine learning algorithm to predict the motion state of the patient, wherein the at least one machine learning algorithm was trained on the basis of; at least one sensor data of one or more reference patients undergoing one or more reference MRI scans by one or more reference MRI scanners; at least one scan parameter of the reference MRI scanners used for the one or more reference MRI scans.

In an example, the at least one machine learning algorithm was trained on the basis of; at least one characteristic of each of the reference patients.

In an example, the machine learning algorithm comprises one or more neural networks.

In an example, the machine learning algorithm comprises one or more classical machine learning algorithms.

In an example, the machine learning algorithm comprises one or more Support Vector Machine (SVM).

In an example, the machine learning algorithm comprises one or more decision Trees.

In an example, the at least one machine learning algorithm comprises two parts. A first part of the machine learning algorithm is used to determine a movement state of a person. Here movement state can mean a likelihood of moving, which can have different levels even for a stationary patient. Thus, a stationary patient can be determined to have a low likelihood of moving, or a stationary patient can be determined to have a high likelihood of moving. A second part of the machine learning algorithm can then operate to determine a prediction into the future of the movement state of the person. The first part can be for example be a standard neural network such as a convolutional neural network (CNN), and the second part can be a recurrent neural network (RNN) or long short-term memory (LSTM) neural network version of a RNN. Thus, here neural network can refer to a combination of neural networks.

In an example, the at least one sensor data of the patient was acquired by one or more of: camera, microphone, skin resistance sensor, skin temperature sensor, skin humidity sensor, skin accelerometer, skin colour sensor, skin conduction sensor, pulse sensor, breathing sensor, Oxygen saturation sensor, radio frequency radar sensor, EEG sensor, ECG sensor, pressure sensor, weight sensor.

In an example, the at least one sensor data of the one or more reference patients was acquired by one or more of: camera, microphone, skin resistance sensor, skin temperature sensor, skin humidity sensor, skin accelerometer, skin colour sensor, skin conduction sensor, pulse sensor, breathing sensor, Oxygen saturation sensor, radio frequency radar sensor, EEG sensor, ECG sensor, pressure sensor, weight sensor.

In an example, the at least one sensor data of the patient comprises one or more of; breathing rate data, heart rate data, voice data, skin resistance data, skin temperature data, skin humidity data, skin motion data, skin colour data, skin conductance data, body part movement data, blinking frequency data, EEG data, ECG data, breathing data, blood oxygen level data, information relating to what is being shown on an in-bore display, weight distribution on in-bore examination table.

In an example, the at least one sensor data of the one or more reference patients comprises one or more of; breathing rate data, heart rate data, voice data, skin resistance data, skin temperature data, skin humidity data, skin motion data, skin colour data, skin conductance data, body part movement data, blinking frequency data, EEG data, ECG data, breathing data, blood oxygen level data, information relating to what is being shown on an in-bore display, weight distribution on in-bore examination table.

In an example, the at least one scan parameter of the MRI scanner comprises one or more of: duration of scan, duration of scan remaining, current gradient strengths, future gradient strengths, type of contrast, timing parameters, SAR (RF-settings), k-space sampling pattern, coil or coils used (e.g., knee coil, head coil, etc.), number of averages, EPI factor, resolution, scan plane, motion direction, anatomic region being scanned.

In an example, the at least one scan parameter of the reference MRI scanners comprises one or more of: duration of scan, duration of scan remaining, current gradient strengths, future gradient strengths, type of contrast, timing parameters, SAR (RF-settings), k-space sampling pattern, coil or coils used (e.g., knee coil, head coil, etc.), number of averages, EPI factor, resolution, scan plane, motion direction, anatomic region being scanned.

In an example, the at least one characteristic of each of the reference patient comprises one or more of: prior movement data for the patient, age, weight, body-mass index, information on a previous diagnosis, physical condition of the patient, psychological condition of the patient, completed questionnaire information.

In an example, training of the machine learning algorithm comprises utilizing information regarding one or more previous MRI scans undertaken by at least one reference patient of the one or more reference patients.

In an example, step d) comprises determining by the processing unit if the predicted motion state of the patient will exceed a motion threshold level, and wherein the prediction if an intervention to the MRI scan and/or MRI scanner and/or patient and/or operator of the MRI scanner is required comprises a determination if a movement level of the patient is predicted to exceed the threshold.

Thus the threshold level can be set below that when movement will compromise imaged quality, and as the patient's movements approach the image degradation threshold the movement threshold will be reached first and the patient can be warned to stop moving before image degradation occurs.

In an example, the motion threshold level is determined on the basis of at least one remaining scan sequence for the MRI scan.

The apparatus for monitoring of a patient undergoing a Magnetic Resonance Image scan, the imaging system, and the method for monitoring of a patient undergoing a Magnetic Resonance Image scan as described with respects to Figs. 6-8 are now described in further detail with respect to specific embodiments, where reference is made to Figs. 9-13. Here, specific relevance relates to intervening in the scan, to enable the scan to continue, by preventing motion of the patient or stress of the patient to becomes problematic. Thus, an intervention can be made that intends to reduce the impact of the predicted anxiety and movement levels if high, and can include staff guidance in adapting the examination for example. In summary, data gathered before and during the current MRI exam is used to predict the level of anxiety (stress state) and movement (motion state) of a patient. In determining this, similar data from other patients (historic data) can be utilized. On the basis of the predicted levels of anxiety and movement, a range of interventions can be done or proposed with the goal to provide guidance to the patient, and/or reassure the patient and optimize MR image quality.

Fig. 9 shows a high-level system architecture. A patient is undergoing an MRI scan, and biometric and physiological sensors and motion snesors indicated at "A" provide corresponding data to the processing unit indicated out "PU". Scan parameters for the MRI scan, indicated at "B", are also provided to the processing unit. These relate to the scan sequence that has already been applied and that to be applied, which therefore provides information relating to how disturbing the scan has been, that can be correlated with sensor data, and how disturbing the scan will be that be used in predicting when to intervene in the scan. Therefore, here the structure of the ExamCard can also be provided to the processing unit, including information such as how long the complete exam or scan will take and how much time is remaining. Information relating to the patient, indicated at "C", is also provided to the processing unit. Labels for training, such as patient feedback for this patient and other patients, past scan information for this patient and other patients, information relating to whether the patient or other patients have pressed the emergency stop button and when, and self-evaluation information relating to for example how stressed the patient or other patients feel or felt is provided at "L". The processing unit implements a first neural network, indicated at "D", such as a convolutional neural network to predict a stress level for the patient and predict of movement state level of the patient. The processing unit also implements a second new network, indicated at "E", such as a recurrent neural network or Long Short term memory comes new network that acts to predict into the future the stress level and movement state level of the patient. The current and predicted stress level is then displayed to the operator, indicated at "F", and decision logic indicated at "G", utilises the predicted stress level and the predicted movement state level to determine if an intervention to the patient or scan should be made, indicated at "H". Thus, the processing unit predicts the patient's stress level and movement state based on a number of parameters ("features") obtained from sensors, scan settings, and patient information. Patient feedback on the experienced stress and movement for that patient and other patients can also be provided for training the internal machine learning algorithms, which can be combined with sensor data and scan parameter information and patient information for those patients undergoing those scans as part of that training.

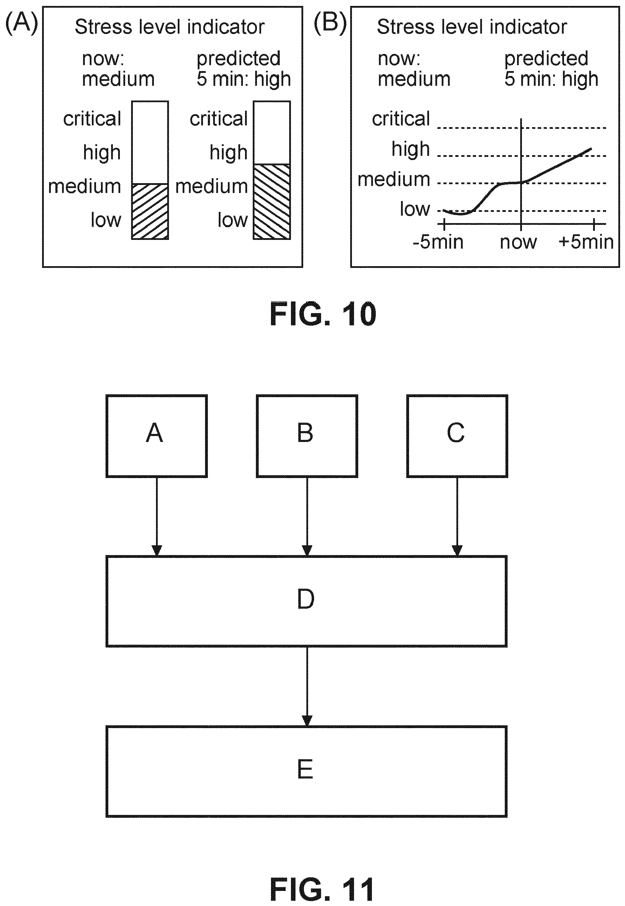

Fig. 10 shows two examples of motion level indicators determined by the processing unit that are presented to the operator or technologist operating the MRI scanner, indicating the present motion level and future motion level and how that motion level has been progressing up to now and how it is predicted to progress into the future. Stress (or anxiety) state level indicators, indicating the of stress level of the patient as of now, in the past, and into the future can also be provided in a similar manner as discussed above. The motion state or level information and/or stress state level information enables the technologist or operator to initiate an intervention if required, and the system itself also can initiate the intervention if it is predicted that the stress level will go too high or the likelihood of the patient moving is too great.

The detection logic operates based on the current and predicted stress level and on the current and predicted movement state level information, where the detection logic decides when to intervene in the scan or to the patient. Ideally, the logic is configured in a way that an intervention is made when an increase of stress level above the critical level is expected while the scan is running, or when the likelihood of the patient moving to an unacceptable level is expected, which would mean that the patient would otherwise press the emergency button with a high probability or move in a manner that would compromise scanning image integrity or result in a potentially dangerous situation arising. Thus, the scan can continue.

### Further details regarding the possible inputs to the processing unit are:

### Sensors for emotional and physical state of the patient can include:

Camera-based heart rate and breathing detection. This can be used to measure instantaneous heart rate, which increases when anxiety increases. The heart/breathing rate measured with the preparation app at home can serve as a baseline for this (see below for details of the preparation app).
Camera-based face recognition and emotional state determination (the skilled person would appreciate that this is available AI technology)
Camera-based movement detection technology
Microphone for speech and voice emotion recognition (the skilled person would appreciate that this is available AI technology)
Electrodermal activity sensor
Skin resistance sensor
Skin temperature sensor
Skin humidity sensor
Skin accelerometer
Other pulse and breathing sensors
Camera-based motion detection
Camera-based blinking frequency detection
RF Pilot tone
RF Radar sensor
EEG and ECG data
Hand movement sensor, where for example this can establish micromotion or minimal motion of the hand, which can be unconscious, can link to tension in the hand indicating a rise in stress level
Additional or complementary physiological or psychological measures can be taken that can be reflective of increasing anxiety and discomfort: and which include heart rate, respiration frequency, electrodermal activity
Monitoring the weight distribution on the patient table. It is established that if a patient is beginning to feel that a body part is about to move involuntarily, they can tense up the whole body or major parts of the body to compensate for this impending movement and this can be detected
Sedation monitoring via vital sign monitoring to determine when sedated patients start to wake up. It has been determined that as a patient starts to wake up this can lead to anxiety and motion

### Scan parameter information can include:

Type of contrast (T1, T2, DWI, etc...)
Timing parameters
Gradient strength settings
SAR (RF-settings)
k-space sampling pattern
Remaining scan time
Information on what is being shown on an in-bore display to the patient, because it has been established that what is shown to the patient can influence their stress level and movement state.

### Patient information (e.g., from electronic medical record) can include:

Age
Weight
Body-mass-index
Previous diagnosis
Other physical and psychological conditions
From data obtained in the preparation room or at home, before the scan (camera, interview, questionnaire)
more information on such **preparation data** is detailed below.

Thus, in summary a prediction of the stress level and movement state level is realized by an artificial intelligence (AI) algorithm, using the features as input and the stress level/movement state level as output. The algorithm can be a combination of a machine learning approach for the derivation of the current stress level and movement state level (such as support vector machine or neural network), and a machine learning approach for predicting the development of the stress level during the next few minutes (such as RNN or LSTM). Supervised training of the AI algorithm can be realized using feedback from the patient and other patients (e.g., emergency button status or self-estimation of stress level) as labels, and via associated sensor data, scan parameter data for those scans and patient information for the other patients for those scans. Training the algorithm on patient feedback allows to normalize the stress level/movement state level to an individual severity scale. This means that a scale can be defined as a number in the range from 0 (low stress level/low movement state level - i.e., low movement likelihood) to 1 (critical stress level/critical movement state level - i.e., high movement likelihood), where reaching the critical stress level means that the patient is very likely to press the emergency stop button and where reaching the critical movement state level means that the patient is very likely to move and compromise the scan. The output of the processing unit is a value for the patient's current stress level and a prediction of the evaluation of this stress value over the next few minutes, and a value for the patient's current movement state level and a prediction of the evaluation of this likelihood of movement value over the next few minutes. The output of the processing unit can be provided to an indicator display for the technologist and to an emergency decision logic. In this manner, a determination can be made to stop the scan, or provide appropriate intervention to continue with the scan,

It is to be noted that in general not all of the abovementioned data will be available for each individual patient. However, the data that are available can be used as input for an anxiety & movement-prediction algorithm. On the basis of the data gathered for the current MRI exam, data on the patient, as well as on the basis of the data of potential earlier exams if available, a prediction can be made as to the expected level of anxiety and likelihood of movement. In addition, on the basis of generic as well as individual data of earlier scans, a prediction can be made of expected image quality (movement interference) and its relation to anxiety/likelihood of movement. Thus, the scan can be stopped before movement adversely affects the quality of the scan, or an appropriate intervention can be made to enable the scan to continue through guidance being provided to the patient, reassurance being provides the patient, or through an appropriate reordering of scan steps.

Fig. 11 provides an overview of the data types and flow utilised in determining stress state and motion state, enabling both an appropriate termination of the scan and/or an intervention to be made. In Fig. 11 box "A" represents data from electronic medical records, for example patient disease history; patient age, number of earlier scans, anxiety during those scans, and efficiency of those scans. Box "B" Preparation data, for example the number and type of training exercises completed, heart rate and breathing rate at home, how long a person is able to lie still. Box "C" represents in-bore measurements, which are outlined in detail above, in which example can include heart rate and breathing rate data, and limb movement data. At box "D" the following is measured/determined: anxiety and movement, and a prediction is made of the likelihood that the patient will abort the scanner (incompletion chance), or the likelihood that the patient will move in a manner that would compromise scanning integrity. At box "E", an appropriate intervention is made: for example, trigger staff to reassure patients, adapt in-bore experience (e.g., additional distraction/guidance), adapt exam cards/scan parameters to increase the chance that the patient will be successfully be able to complete the examination (e.g., shorten the exam card; change the order of sequences, etc).

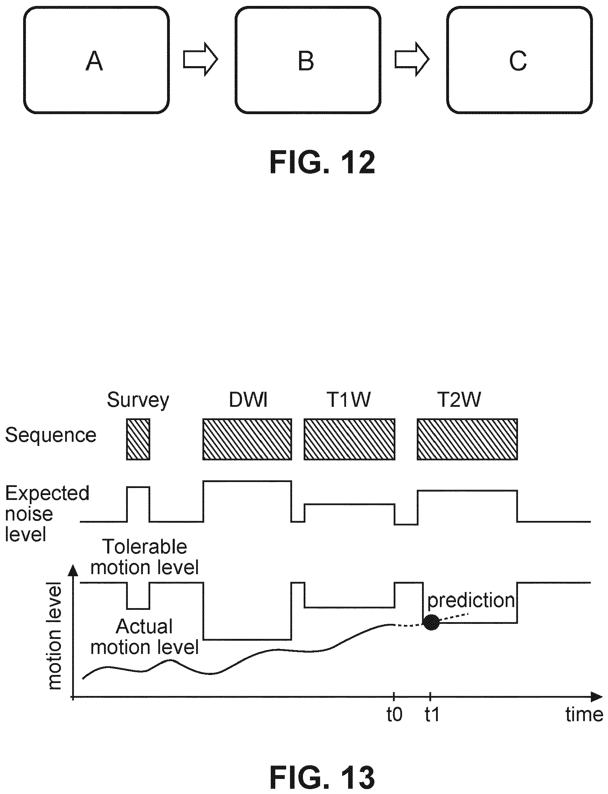

Fig. 12 shows a high level architecture of a detailed example of an apparatus for monitoring of a patient undergoing a Magnetic Resonance Image (MRI) scan. At "A" motion sensor data is acquired during for example a survey scan to form baseline motion data, and during the actual scan itself motion data of the patient is detected and acquired. All of this information, along with information such as data relating to the patient, and data relating to the scan already being undertaken and that to be undertaken is provided to an algorithm indicated at "B", that defines the best strategy for providing an intervention to the patient, providing intervention information to an operator, automatically providing intervention to the scan itself, detailed at "C".

Thus, in an example of how the apparatus can operate, a motion detector is used to create a baseline of motion during the survey scan. This information is used in an algorithm to set a certain threshold and calculates how it will affect the images of the remaining sequences in the Examcard. Based on this a certain feedback is giving to the technologist ranging from repositioning the patient to get more comfort, stabilize the patient more or some coaching advice. Towards the patient it will be communicated that certain motion was detected and that he/she will be notified if certain motion is detected during the scan (this can be communicated in a more specific way by mentioning the body part), e.g., using a simple traffic light. When a sequence is more motion sensitive the threshold for feedback will be lowered for that sequence. If motion becomes more severe new advice can be given to technologist to change certain image parameters or to motivate the patient extra and shifting the most sensitive sequences to the beginning of the Examcard.

Fig. 13 shows a representation of an MR sequence, pre-defined tolerable motion levels, and measured actual and predicted motion level as functions of time. Here, the motion level of the patient is monitored continuously. The motion level can be calculated as the first derivative of any position measurement, or as a temporal moving average thereof. At any point in time, a prediction of the patient's motion level in the next time period (several seconds or minutes) is calculated using the AI algorithm (as detailed here). The predicted motion level is compared with a pre-defined tolerable motion level that depends on the exact settings of the MR sequence. The tolerable motion level can be different for different image acquisition settings. If the patient's motion is predicted to exceed the tolerable motion level within the following time period, a warning or and advice to the patient can be given to lower the motion level. Therefore, in the example shown here at the current time t0, the motion level is still tolerable (below the tolerable level). However, the prediction shows that at time t1, the motion level will probably be too high (indicated by the orange circle). An alert or advice to the patient (intervention) can be given to prevent this from happening.

The AI algorithm to predict the motion level is similar to that described above, and can be implemented in terms of a recurrent neural network (RNN), preferably as a more advanced Long-Short-Term-Memory (LSTM) network. An input vector of fixed size can be supplied to the LSTM cells at any time point. This vector consists of: The tolerable motion level, The patient's actual motion level. Optional inputs can be: The expected noise level or psychoacoustic features of the sequence, Patient characteristics (age, weight, medical condition), Patient's likelihood to move known from previous examinations. The patient's motion level of the next time step(s) is then an output parameter of the network. The optional parameters described here that can be included in the feature vector, enable a more accurate the prediction of when to intervene to be made. The network can be trained on full data sets of previous examinations.

Thus, the expected sequence timing and machine idle times may be adapted dynamically as the operator pauses or changes the sequences.

### Intervention options

The predicted anxiety and movement levels can be used in several ways to optimize the quality of the MRI exams:
Based on preparation and historic data, the level of anxiety of the patient *at the start* of the MRI exam can be predicted. In case of high levels, hospital staff can be advised to take ample time to reassure the patient, or to talk to the patient in a certain way to reassure the patient. Additionally, the system can automatically be adapted to reduce patient anxiety levels (e.g., by providing more distracting in-bore entertainment at crucial moments), or by providing the patient with a chance to temporarily pause the scan; or by adapting the exam card (e.g., by shortening the scan if the patient is not expected to complete the full examination).

Based on preparation, historic data, and in-bore data, the *current* level of anxiety of the patient can be estimated. In case of high levels, hospital staff can be triggered to pay additional attention to the patient, or to talk to the patient in a certain way to reassure the patient. Additionally, the system can automatically be adapted to reduce patient anxiety levels (e.g., by providing more distracting in-bore entertainment at crucial moments), or by provide the patient with a chance to temporarily pause the scan; or by adapting the exam card (e.g., by shortening the scan if the patient is not expected to complete the full examination). In addition, it is possible to reschedule the order of the various sub-exams, such that sub-exams that are less sensitive to anxiety and motion interference are scheduled first.

Based on preparation, historic data, and in-bore data, the *upcoming* levels of anxiety of the patient can be predicted for a number of minutes ahead. This allows rescheduling of the sub-exam order such that the negative impact of anxiety and motion interference are minimized and kept at acceptable levels during the exam. In case of high levels, hospital staff can be triggered to pay additional attention to the patient, or to talk to the patient in a certain way to reassure the patient. Additionally, the system can automatically be adapted to reduce patient anxiety levels (e.g., by providing more distracting in-bore entertainment at crucial moments), or by provide the patient with a chance to temporarily pause the scan; or by adapting the exam card (e.g., by shortening the scan if the patient is not expected to complete the full examination). In addition, it is possible to reschedule the order of the various sub-exams, such that sub-exams that are less sensitive to anxiety and motion interference are scheduled first.

In addition, the predicted data of *all patients* scheduled for a certain day can be used to devise the optimal assignment of MRI machine and hospital staff (for instance those experienced in reassuring anxious patients). In principle this assignment schedule can be prepared at the start of the day on the basis of preparation and historic data. In addition, the schedule can be optimized during the day on the basis of the incoming in-bore data.

### The following details an exemplar process or how this can proceed:

Three steps can be involved in this order:
The preparation data is analysed before patients have their scan, and the results (and recommendations) are presented to the staff/techs before they meet the patients (i.e. pick them up from the waiting area).

The data is used to profile the patient in one of several intervention categories:
No intervention: the patient has indicated in questionnaires to be able to have the scan without difficulties (e.g. not claustrophobic, no sensitive hearing, low excitability, low nervousness etc). AND the recorded data from app has shown good results (good patient understanding and skill performance) AND the HR data is stable and in a good zone (e.g. HR between 60-90 bpm, and less than 20-30 bpm more in the waiting room than when measured earlier at home).

Skills intervention: the patient has a high probability to have a successful scan, but lying still needs to be trained further and/or breath holding.

Anxiety management: the patient has indicated that there is high level of anxiety despite good results with the exercises (meaning that the skill level is sufficient). Interventions should be geared towards understanding the biggest source of anxiety and exercises addressing this.

Overall training: the patient has high anxiety and low skill level. Interventions are a combination of ii and iii. In addition, these patients will receive special attention before, during and after the scan.

An appropriate self-use module, can be utilized by patients in the home environment on for example a mobile app.

Identification of the practiced skills and/or of the sources of anxiety though the performed exercises and self-reported data.

Additional modules will be offered and scheduled for patients with skills (a number of lying still exercises and breath holds). Progress will be monitored. If skills improve, this will be evident in the report for the radiologist.

Additional modules will be offered towards anxiety management depending on the biggest stressors: e.g. relaxation exercises, desentization exercises, coping skills training.

On-site support: The report to radiologists/technologist will indicate the results of the patient preparation and interventions. Based on the report, a number of recommendations can be provided to the radiologist/technologist.

Patient will be fine. No problem expected. Keep positive and relaxed with patient.

Patient practiced skills at home and passed. Address this and get patients thoughts on it ('Breath holds proved a little difficult, but you managed with practice! Let see how this goes, and we'll try to give you some extra time and tips for those sessions')

Patient practiced anxiety management at home and passed. Address this, and make sure that communication is ongoing with patient. Monitor HR in-bore carefully and schedule more time. Patient may need to be retracted from bore to breath and calmed down once or twice during the scan.

Patient practiced skill but did not succeed. Address this and plan for the right scanning sequences. Also real-time instructions and feedback in-bore may help (only if anxiety is low) ('You're doing well, keep you breath in a for a few seconds longer.' 'You're still moving your head a bit. Stay still for another minute and then we can do some finger and toe wiggling before we start the next sequence!');
In some cases it may be decided to subject the patient to sedation/medication, or refer to a specialized imaging centre.

In addition, heart rate and breathing measurements can indicate increases in in-bore anxiety. Differences as large as 20-30 bmp have been observed as a consequence of waxing and waning anxiety.

The following provides an overview understanding of the developments made leading to the described techniques:
In seeking to improve MRI scans, the inventors realised that the two major limitations were stress developing in the patient during the scan and movement of the patient. In researching these areas the inventors establish that movement and stress are only weakly related (and in some studies, they are completely uncorrelated). For example, extremely anxious patients may show very little movement in the scanner (they are too "frozen" or "petrified" to move). And relaxed patients may move because they have an itch/because a body part falls asleep or they are so relaxed about the procedure that they forget they shouldn't move.

Further examples are:
A sudden reduction of movement in a body part may increase the risk of motion, while the anxiety-curve may show a very different pattern.

Thus, if a person's arm falls asleep, this may temporarily reduce motion in that arm in the short term, but increase the likelihood of motion in the longer term (once the numbness turns into pain and the person repositions)..

If a person completely falls asleep, there will be very little motion until that person wakes up. How long the person has been asleep and how deep their sleep is and when they are predicted to wake (combined with MR noise level etc) can be a better predictor of movement in the future.

Thus, this means that there is a complex relationship between anxiety & movement and that it is possible that reducing anxiety can either increase motion likelihood (e.g., when going from a "petrified" state to a slightly less anxious state) or reduce it, depending on a number of factors.

However, the inventors established that by monitoring the patient with sensors, and using scan parameters of the scan now and in the future, and information relating to the patient it is possible to train machine learning algorithms to determine both how a stress level will develop and how the likelihood of the patient moving will develop.

Thus, the inventors were aware of AI-based tools that utilize sensor information for recognizing human emotions that have been developed in recent years. This includes facial emotion recognition (e.g., https://azure.microsoft.com/en-us/services/cognitive-services/emotion/), speech emotion recognition (e.g., http://www.good-vibrations.nl/, https://vokaturi.com/), and emotion recognition though other physiological sensors (see, e.g., https://www.wareable.com/health-and-wellbeing/future-of-emotion-sensing-wearables-111, https://biosay.com/). The inventors utilized the understanding of these technologies in combination with scan information of a MRI scanner, patient background information and additional sensor in combination with a dedicated prediction algorithm, to help detect and react to emergency situations in MR examinations and increase patient comfort and safety. In this manner. The the emotional and physiological state of the patient is in effect analysed and the development of this state is predicted. Real-time feedback is provided to the technologist and an automatic decision can be made when to stop a scan based on the current and predicted emotional and physiological state of the patient. In this way, patient experience is improved (less anxiety or pain), the patient is safer due to an automated emergency stop of the system, and the workflow can be improved, because scans can be stopped and repeated earlier.

Data gathered before the current MRI exam is utilised in determining the predicted stress level and predicted motion state level as discussed above. Below, is described in detail one specific methodology by which this information, or at least part of this information relating to the patient, can be gathered. The data gathered is termed "preparation data".

### Preparation data

Before the MRI-exam, patients receive online information and training (e.g., in the form of a smartphone app) to help them prepare for the upcoming MRI-exam at home or within the hospital. This app can consist of several elements, such as a) information for patients; b) training exercises; c) questionnaires about the patient; and d) a measurement of their heart and breathing rate, skin colour, and/or reaction speed.

The information for patients can consist of text, video, or audio that gives patients information about the upcoming scan or medical procedure (e.g., a video with information about an MRI-scan, the MRI machine, and what happens).

The training exercises provide participants with specific coaching or training to improve skills that allow them to undergo the medical procedure; among other things, exercises include improving the ability to lie still for an extended period of time and/or the ability to hold their breath for a certain time/follow a specific breathing pattern.

Questionnaires will consist of demographic questions, e.g., about a patient's age, number of previous scans, educational level, etc; as well as other relevant questions (e.g., about patient's claustrophobia, anxiety level, etc.). In addition, other questionnaire input like self-reports can be gathered. For example, patient's perception of the upcoming scan and their own expectation. Finally, each piece of information or activity can be followed by questions related to the patients' perception of the scan.

The heart and breathing rate measurement can be acquired with the aid of a mobile phone. It can give an indication of someone's basal heart rate, possibly before and after the training material & questionnaires were accessed by the user. Such measurement can also be followed up by the patient's perception and expectation. Heart rate and breathing rate measurements can also be conducted just before the examination and compared to the baseline measurements done earlier.

Skin colour could be determined with a phone in the face area as measured before the examination and just before the examination.

By creating a small game-app, patient's reaction time can be determined at home and when they are sitting in the waiting room.

As such, the preparation data can consist of:
Usage data from a mobile application (e.g., number of logins, time spent in the app);
Data from questionnaires (e.g., questionnaires about the patient's age, educational level, anxiety, and perception and expectation etc.);
Training-specific data (e.g., the amount of time a participant is able to lie still when training at home; the number of seconds they can hold their breath; their heart rate as measured by the app, the number of times they use the different features of the app). Physiological parameters (heart rate or breathing rate changes, skin colour change, and reaction time difference).

### Training of the Machine Learning Algorithm

Regarding the implementation of the one or more machine learning algorithms, as discussed above there are two parts of the implementation that can be distinguished:
Machine learning algorithms (classical SVM, decision trees, or neural networks) can be employed to determine the current stress level and determine the current movement state level from sensor data and other information (not yet predicting the future). In this case, the algorithm would be trained with historic data that is labelled using subjective feedback from the patient or objective measurements, such as frequency of patient-initiated scan aborts or severe motion artifacts, or other information as discussed above. Alternatively, the stress level and/or movement state level can be computed from the sensor data using an analytic known function.

For the prediction of the future development of the stress level and of the future development of the movement state level, an implementation using recurrent neural networks (RNNs) has been found to be particularly suitable. Specifically, a Long-Short-Term-Memory (LSTM) implementation has been found to be well suited here. This machine learning implementation is fed with both context information (patient condition, type of scan, etc.) and the development of the stress level as a function of time (up to the present moment) and/or movement state level as a function of time. The network would then predict how the stress level will continue in the future and how the movement state level will continue into the future. To train such a network, it is necessary to record the temporal evolution of the stress level, and movement state level, along with the context information for multiple subjects and use that as training data.

It is to be noted, that the two parts discussed above can operate individually for stress level and movement state level prediction, or combined. Thus, there can be algorithms at 1 that determine the current stress level and different algorithms that determine the current movement state level. Then at 2, there can be there can be algorithms that predict the future stress level and different algorithms that predict the future movement state level. However, the same algorithms at 1 can determine both stress level and movement state level, and the same algorithms at 2 can predict the future stress level and movement state level,

### Real-Use Implementations

The following provides details on various embodiments that can be provided, on the basis of combinations of various measurement options:

### Embodiment 1: full implementation

The above full-fledged description combines preparation data, in-bore data, and historic data, and allows to give anxiety reassurance at start and during the exam, as well as optimal scheduling of the present exam as well as of the other exams (of other patients) that day.

### mbodiment 2: using only (historic) preparation data

The use of only preparation data (potentially in combination with the preparation data of earlier exams) allows an estimate of the upcoming anxiety level of the patient. This can be used to schedule and assign all exams of the day to suitable rooms and hospital staff with experience with anxious patients if needed. This also allows the staff to spend more time reassuring the patient just before the MRI exam (dividing patients over the "care lane", if they are expected to be anxious, and the "fast lane", if they are expected to value efficiency). If the app also gives information whether the patient has potentially arrived early in the hospital (many patients do that), their presence can be used as input to the rescheduling.

### Embodiment 3: using only (historic) in-bore data

The use of only (historic) in-bore data allows the estimation and prediction into the near future of a patient's anxiety and stress levels. These can be used for suggesting the hospital staff to reassure the patient as well as for scheduling the MRI sub-exams such that optimal MRI quality can be achieved.

Therefore, in an exemplar apparatus, a patient undergoes an MRI scan, and sensor data can be used to measure body motion, derived from for example: MRI signal, Camera (e.g., VitalEye), breathing belt, laser, accelerometer or other sensor placed on the patient. To determine the type of feedback and the optimal moment of feedback, an algorithm gets input from the motion sensor(s) and/or MR sequence parameters, progress of the acquisition, Examcard parameters, patient physiological parameters (ECG, breathing rate, O2-saturation, pressure sensors in the patient table, skin discolouring or skin conduction (GSR)). The discomfort level of the patient can be quantified by measuring O2-saturation level of a specific arm or leg and/or detecting changes in the pressure distribution (to assess if the body is compensating for the discomfort by muscle tension). The algorithm uses AI to predict the motion or moment of motion upfront based on the previous described input data. The prediction is used to refine the decision of feedback type and moment. The feedback can be given by in-bore visible screen, via a tactile sensation, audible or lights (color, tone, intensity).

Therefore, in summary the apparatus, system and method described here mitigates the consequences of the many different types of motion that can occur during an examination, ranging from rigid/deformable motion, repetitive/random, and different directions (in-plane, out of plane, rotation) and/or amplitude (see for example Zaitsev, M., Maclaren, J., & Herbst, M. (2015). Motion artifacts in MRI: a complex problem with many partial solutions. Journal of Magnetic Resonance Imaging, 42(4), 887-901.) Some of these motions can be compensated within a MR scan or between different MR scans in an Examcard, but it is not easy and requires a longer examination or reconstruction duration, and data can be removed from a final scan that has motion artefacts, but leads to a reduced dataset. The inventors realised that preventing motion would lead to better outcomes than compensating for it (after the fact), and this led to the development of the ability to intervene in the scan as described above. Also, the scan can be stopped if necessary, meaning that the dataset does not have motion artefacts, but by intervening the inventors have established that a full dataset can be acquired. The inventors started from an understanding that motion can occur voluntary or involuntary, but in most cases the patient can influence this. The problem is that the patients, currently, are not aware of their own body motion and/or do not know that the moment of motion can be critical for the diagnostic value of the acquired image. However, through utilization of the apparatus, system and method described here the right feedback and moment of feedback can be provided to the patient, and patients become better able to control their behaviour and, in so doing, contribute to a successful examination. However, providing patients with feedback that is too frequent, or that isn't optimally matched to their movement, can lead to irritation, feelings of insecurity, and as such the intervention is provided only when necessary. Thus, with this intervention and feedback, the need for re-scans can be reduced and patients feel more engaged in their own care, which can positively affect their experience and distract them from rumination, negative thoughts, pain, or physical discomfort.

In another exemplary embodiment, a computer program or computer program element is provided that is characterized by being configured to execute the method steps of the method according to one of the preceding embodiments, on an appropriate apparatus or system.

The computer program element might therefore be stored on a computer unit, which might also be part of an embodiment. This computing unit may be configured to perform or induce performing of the steps of the method described above. Moreover, it may be configured to operate the components of the above described apparatus and/or system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An apparatus (10a) for monitoring of a patient undergoing a Magnetic Resonance Image (MRI) scan, the apparatus comprising:
- an input unit (20a);
- a processing unit (30a); and
- an output unit (40a);
wherein, the input unit is configured to provide the processing unit with at least one sensor data of a patient undergoing an MRI scan by an MRI scanner;
wherein, the input unit is configured to provide the processing unit with at least one scan parameter of the MRI scanner for the MRI scan;
wherein, the processing unit is configured to predict if an intervention to the MRI scan and/or MRI scanner and/or patient and/or operator of the MRI scanner is required, the prediction comprising utilization of the at least one sensor data of the patient, and the at least one scan parameter of the MRI scanner; and
wherein, the output unit is configured to output information relating to the prediction of the intervention.

2. Apparatus according to claim 1, wherein, the input unit is configured to provide the processing unit with at least one characteristic of the patient; and wherein the prediction if an intervention to the MRI scan and/or MRI scanner and/or patient and/or operator of the MRI scanner is required comprises a prediction of a motion state of the patient, and wherein the prediction of the motion state comprises utilization of the at least one sensor data of the patient, the at least one scan parameter of the MRI scanner, and the at least one characteristic of the patient.

3. Apparatus according to claim 2, wherein the at least one characteristic of the patient comprises one or more of: prior movement data for the patient, age, weight, body-mass index, information on a previous diagnosis, physical condition of the patient, psychological condition of the patient, completed questionnaire information, patient feedback.

4. Apparatus according to claim 3, wherein the prior movement data for the patient comprises information regarding one or more previous MRI scans undertaken by the patient.

5. Apparatus according to any of claims 2-4, wherein the processing unit is configured to implement at least one machine learning algorithm to predict the motion state of the patient, wherein the at least one machine learning algorithm was trained on the basis of; at least one sensor data of one or more reference patients undergoing one or more reference MRI scans by one or more reference MRI scanners; at least one scan parameter of the reference MRI scanners used for the one or more reference MRI scans.

6. Apparatus according to any of claims 1-5, wherein the at least one sensor data of the patient was acquired by one or more of: camera, microphone, skin resistance sensor, skin temperature sensor, skin humidity sensor, skin accelerometer, skin colour sensor, skin conduction sensor, pulse sensor, breathing sensor, Oxygen saturation sensor, radio frequency radar sensor, EEG sensor, ECG sensor, pressure sensor, weight sensor.

7. Apparatus according to claims 5-6 wherein the at least one sensor data of the one or more reference patients was acquired by one or more of: camera, microphone, skin resistance sensor, skin temperature sensor, skin humidity sensor, skin accelerometer, skin colour sensor, skin conduction sensor, pulse sensor, breathing sensor, Oxygen saturation sensor, radio frequency radar sensor, EEG sensor, ECG sensor, pressure sensor, weight sensor.

8. Apparatus according to any of claims 1-7, wherein the at least one scan parameter of the MRI scanner comprises one or more of: duration of scan, duration of scan remaining, current gradient strengths, future gradient strengths, type of contrast, timing parameters, SAR (RF-settings), k-space sampling pattern, coil or coils used, number of averages, EPI factor, resolution, scan plane, motion direction, anatomic region being scanned.

9. Apparatus according to any of claims 5-8, wherein the at least one scan parameter of the reference MRI scanners comprises one or more of: duration of scan, duration of scan remaining, current gradient strengths, future gradient strengths, type of contrast, timing parameters, SAR (RF-settings), k-space sampling pattern, coil or coils used (e.g., knee coil, head coil, etc.), number of averages, EPI factor, resolution, scan plane, motion direction, anatomic region being scanned.

10. Apparatus according to any of claim 5-9, wherein the at least one characteristic of each of the reference patient comprises one or more of: prior movement data for the patient, age, weight, body-mass index, information on a previous diagnosis, physical condition of the patient, psychological condition of the patient, completed questionnaire information.

11. Apparatus according to any of claims 5-10, wherein training of the machine learning algorithm comprises utilization of information regarding one or more previous MRI scans undertaken by at least one reference patient of the one or more reference patients.

12. Apparatus according to any of claims 2-11, wherein the processing unit is configured to determine if the predicted motion state of the patient will exceed a motion threshold level, and wherein the prediction if an intervention to the MRI scan and/or MRI scanner and/or patient and/or operator of the MRI scanner is required comprises a determination if a movement level of the patient is predicted to exceed the threshold.

13. Apparatus according to claim 12, wherein the motion threshold level is determined on the basis of at least one remaining scan sequence for the MRI scan.

14. An imaging system (100a) comprising;
- a magnetic resonance imaging (MRI) scanner (110a);
- at least one sensor (120a); and
- an apparatus (10a) for monitoring of a patient undergoing a Magnetic Resonance Image (MRI) scan according to any of claims 1-13;
wherein, the at least one sensor is configured to provide the processing unit of the apparatus with at least one sensor data of a patient undergoing an MRI scan by an MRI scanner;
wherein, the apparatus is configured to automatically provide an intervention to the MRI scan and/or MRI scanner and/or patient and/or operator of the MRI scanner on the basis of the information relating to the prediction of the intervention.

15. A method (200a) for monitoring of a patient undergoing a Magnetic Resonance Image (MRI) scan, the method comprising:
- providing (210a) a processing unit with at least one sensor data of a patient undergoing an MRI scan by an MRI scanner;
- providing (220a) the processing unit with at least one scan parameter of the MRI scanner for the MRI scan;
- predicting (230a) by the processing unit if an intervention to the MRI scan and/or MRI scanner and/or patient and/or operator of the MRI scanner is required, the prediction comprising utilization of the at least one sensor data of the patient, and the at least one scan parameter of the MRI scanner; and
- outputting (240a) by an output unit information relating to the prediction of the intervention.

16. A computer program element for controlling an apparatus according to one of claims 1 to 13 and/or system of claim 14, which when executed by a processor is configured to carry out the method of claim 15.
